# EUROPEAN PATENT APPLICATION

(11) **EP 4 628 482 A1**
(43) Date of publication of application: **08.10.2025**
(21) Application number: 23897187.3
(22) Date of filing: 07.09.2023
(51) Int. Cl.: C07D 213/85

(54) **FLUORINE-CONTAINING PYRIDONE COMPOUND AND METHOD FOR PRODUCING SAME**

(30) Priority: 28.11.2022 JP 2022189290
(71) Applicant: Unimatec Co., Ltd., Tokyo 105-0012 (JP)
(72) Inventor: SEINO Junya, Kitaibaraki-shi, Ibaraki 319-1593 (JP); AOTSU Rie, Kitaibaraki-shi, Ibaraki 319-1593 (JP); TAKAHASHI Yusuke, Kitaibaraki-shi, Ibaraki 319-1593 (JP)
(74) Representative: Global IP Europe Patentanwaltskanzlei
(86) International application number: PCT/JP2023/032636
(87) International publication number: WO 2024/116528

(57) **Abstract**

To provide a novel fluorine-containing pyridone compound and a method for producing the same.

A fluorine-containing pyridone compound represented by the following formula (1): 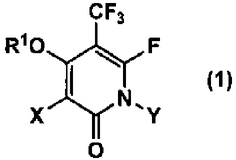 wherein
X represents CO(OₘR²), SOₙ(OₘR²), PO(OₘR²)(OₗR³), CN or NO₂,
Y represents R⁴, OR⁴ or NR⁴R⁵,
R¹ to R⁵ each independently represent a hydrocarbon group having 1 to 12 carbon atoms,
1 and m are each independently 0 or 1, and
n is 1 or 2.

## Description

### Technical Field

The present invention relates to a fluorine-containing pyridone compound and a method for producing the same.

### Background Art

Compounds having a 2-pyridone ring have been known to have various pharmacological actions. Among them, compounds having a 2-pyridone ring with a substituent introduced on the nitrogen atom at the 1-position have particularly excellent pharmacological actions and therefore have been widely used mainly in the fields of pharmaceuticals and agrochemicals.

Examples of a compound having a 2-pyridone ring with a substituent introduced at the 1-position include natural products such as Factumycin and Goldinomycin, which have an inhibitory activity on main protease of severe acute respiratory syndrome coronavirus 2 (SARS-CoV-2), Sambutoxin and Funiculosin, which have an inhibitory activity on ubiquinol-cytochrome c reductase, Heneicomicin and Efrotomycin, which have an inhibitory activity on an elongation factor Tu, Nudifloric acid, which has an inhibitory activity on transcription factors AP-1 and NF-κB, Nudiflorine, which has been suggested to have an antidotal action for organophosphate poisoning, Ricinine, which has been suggested to have an inhibitory activity on casein kinase 1α, and Ricinic acid, which has a cytocidal action on cancer cells.

Moreover, examples of pharmaceuticals include antitumor agents such as Ravoxertinib and Merestinib, an autoimmune disease therapeutic agent such as Fenebrutinib, an antiviral agent such as Doravirine, antifibrotic agents such as Siremadlin and Pirfenidone, an anti-inflammatory agent such as Alvelestat, an antithrombotic agent such as Eribaxaban, an antiepileptic drug such as Perampanel, and antihypertensive agents such as Embusartan, Emakalim, and Bimakalim.

In order to produce a compound having a 2-pyridone ring with a substituent introduced onto the nitrogen atom at the 1-position, an attempt is made to introduce a substituent onto the nitrogen atom at the 1-position of the 2-pyridone ring; however, this introduction may compete with introduction of substituent onto the oxygen atom at the 2-position. This problem particularly becomes pronounced when the substituent to be introduced is a carbon substituent. For example, Non Patent Literatures 1 to 3 have reported a method for introducing a carbon substituent onto the nitrogen atom at the 1-position of a 2-pyridone ring.

### Document List

### Non Patent Literatures

Non Patent Literature 1: Organic Letters, 2021, volume 23, pages 1038-1043
Non Patent Literature 2: The Journal of Organic Chemistry, 2018, Volume 83, Pages 6769-6775
Non Patent Literature 3: Synthesis, 2018, volume 50, pages 1699-1710

### Summary of Invention

### Technical Problem

A fluorine-containing pyridone compound having a substituent on the nitrogen atom at the 1-position and a trifluoromethyl group at the 5-position may be promising not only in the fields of pharmaceuticals and agrochemicals but also in the field of organic electronic materials science, but there are very few examples of such compounds reported. Moreover, in investigating a method for producing such a compound, the method reported in Non Patent Literature 1 limits the substituent to be introduced to a ketomethylene group because a ketosulfoxonium ylide is used, the method reported in Non Patent Literature 2 is considered difficult to introduce an aryl group at the 1-position, and may not be suitable for introducing a substituent having an acid-sensitive site because it involves a rearrangement process taking place by an acidic substance generated in the reaction system, and the method reported in Non Patent Literature 3 may be difficult to quantitively provide a target product depending on a structure of a substituent because the selectivity is highly dependent on a structure of the substituent to be introduced. In view thereof and the like, a new production method with fewer constraints is required.

The present invention provides a novel fluorine-containing pyridone compound and a method for producing the same.

### Solution to Problem

The gist configuration of the present invention is as follows:
**[1]** A fluorine-containing pyridone compound represented by the following formula (1): 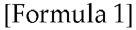 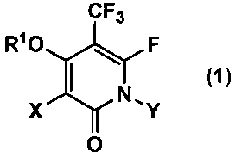 wherein
   X represents CO(OₘR²), SOₙ(OₘR²), PO(OₘR²)(OₗR³), CN or NO₂,
   Y represents R⁴, OR⁴ or NR⁴R⁵,
   R¹ to R⁵ each independently represent a hydrocarbon group having 1 to 12 carbon atoms,
   l and m are each independently 0 or 1, and
   n is 1 or 2.
[2] A fluorine-containing pyridone compound represented by the formula (1) above,
   wherein
   X represents CO(OₘR²), SOₙ(OₘR²), PO(OₘR²)(OₗR³), CN or NO₂,
   Y represents a phenyl group, benzyl group, tolyl group, or naphthyl group to which one or more alkoxy groups having 1 to 5 carbon atoms are optionally bonded,
   R¹ to R³ each independently represent a hydrocarbon group having 1 to 12 carbon atoms,
   1 and m are each independently 0 or 1, and
   n is 1 or 2.
[3] A method for producing a fluorine-containing pyridone compound, having a step of reacting a fluoroisobutylene derivative represented by the following formula (2) with a compound represented by the following formula (3) to obtain a fluorine-containing pyridone compound represented by the following formula (1): 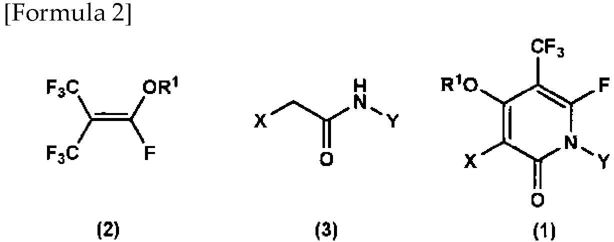 wherein X, Y and R¹ are as defined above.
[4] A method for producing a fluorine-containing pyridone compound, having a step of reacting a fluoroisobutane derivative represented by the following formula (4) with a compound represented by the following formula (3) to obtain a fluorine-containing pyridone compound represented by the following formula (1): 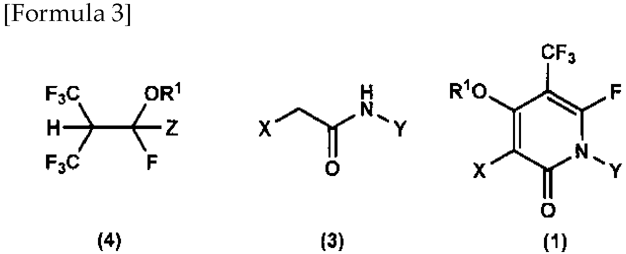
   wherein X, Y and R¹ are as defined above,
   Z represents a halogen atom, OCO(OₖR⁶), or OₖSOᵢ(OⱼR⁶),
   R⁶ represents a hydrocarbon group having 1 to 10 carbon atoms,
   j and k are each independently 0 or 1, and
   i is 1 or 2.

### Effects of Invention

According to the present invention, a novel fluorine-containing pyridone compound and a method for producing the same, can be provided.

### Description of Embodiments

### [Fluorine-containing pyridone compound]

The fluorine-containing pyridone compound of the present invention is represented by the following formula (1).

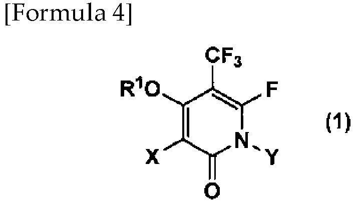

(In the formula (1) above,
X represents CO(OₘR²), SOₙ(OₘR²), PO(OₘR²)(OₗR³), CN or NO₂,
Y represents R⁴, OR⁴ or NR⁴R⁵,
R¹ to R⁵ each independently represent a hydrocarbon group having 1 to 12 carbon atoms,
1 and m are each independently 0 or 1, and
n is 1 or 2.)

The fluorine-containing pyridone compound of the present invention has a substituent on the nitrogen atom at the 1-position and has a trifluoromethyl group at the 5-position. Since a similar pyridone compound having a substituent on the nitrogen atom at the 1-position has been reported to exhibit an excellent activity in the fields of pharmaceuticals and agrochemicals, the fluorine-containing pyridone compound of the present invention is expected to have a similar activity as an alternative. Moreover, the fluorine-containing pyridone compound of the present invention has a plurality of substitution sites that can be further modified (specifically, the 3-, 4-, and 6-positions can be modified), and therefore has high structural expandability. Therefore, the fluorine-containing pyridone compound of the present invention is also promising as an intermediate that can be converted into various highly active compounds.

The hydrocarbon group having 1 to 12 carbon atoms, which R¹ to R⁵ each independently represent, is not particularly limited as long as it is a hydrocarbon group having 1 to 12 carbon atoms and consisting of a carbon atom and a hydrogen atom, and examples thereof include a chain hydrocarbon group, an aromatic hydrocarbon group, and an alicyclic hydrocarbon group. The chain hydrocarbon group is not particularly limited as long as it has a total of 1 to 12 carbon atoms, and may be a linear hydrocarbon group or a branched hydrocarbon group. The aromatic hydrocarbon group is not particularly limited as long as it has a total of 6 to 12 carbon atoms, and may be an aromatic hydrocarbon group having a substituent or an aromatic hydrocarbon group having no substituent. Also, the aromatic hydrocarbon group may have a condensed polycyclic structure. The alicyclic hydrocarbon group is not particularly limited as long as it has a total of 3 to 12 carbon atoms, and may be an alicyclic hydrocarbon group having a substituent or an alicyclic hydrocarbon group having no substituent. The alicyclic hydrocarbon group may also have a bridged ring structure.

Examples of the chain hydrocarbon groups that R¹ to R⁵ represent include alkyl groups such as a methyl group, an ethyl group, a n-propyl group, an isopropyl group, a n-butyl group, an isobutyl group, a s-butyl group, a t-butyl group, a pentyl group, a hexyl group, a heptyl group, an octyl group, a nonyl group, a decyl group, an undecyl group, and a dodecyl group; alkenyl groups such as an ethenyl group, a propenyl group, a butenyl group, a pentenyl group, a hexenyl group, a heptenyl group, an octenyl group, a nonenyl group, a decenyl group, an undecenyl group, and a dodecenyl group; and alkynyl groups such as an ethynyl group, a propynyl group, a butynyl group, a pentynyl group, a hexynyl group, a heptynyl group, an octynyl group, a nonynyl group, a decynyl group, an undecynyl group, and a dodecynyl group.

Examples of the aromatic hydrocarbon groups that R¹ to R⁵ represent include a phenyl group, a benzyl group, a tolyl group, and a naphthyl group. The tolyl group may be any of an o-tolyl group, a m-tolyl group, and a p-tolyl group. To these aromatic hydrocarbon groups, one or more alkoxy groups having 1 to 5 carbon atoms are optionally bonded.

Examples of the alicyclic hydrocarbon groups that R¹ and R⁵ represent include a saturated or unsaturated cyclic hydrocarbon group. Examples of the cyclic hydrocarbon group include a cyclopropyl group, a cyclobutyl group, a cyclohexyl group, a cyclopentyl group, an adamantyl group, and a norbornyl group.

R¹ is preferably a chain hydrocarbon group or an aromatic hydrocarbon group, more preferably a chain hydrocarbon group, still more preferably a chain hydrocarbon group having 1 to 6 carbon atoms, particularly preferably a methyl group, an ethyl group, a n-propyl group, or a n-butyl group, and most preferably a methyl group.

R² and R³ are each independently preferably a chain hydrocarbon group or an aromatic hydrocarbon group, more preferably a chain hydrocarbon group, still more preferably a chain hydrocarbon group having 1 to 6 carbon atoms, particularly preferably a methyl group, an ethyl group, a n-propyl group or a n-butyl group, and most preferably a methyl group.

R⁴ and R⁵ are each independently preferably a chain hydrocarbon group or an aromatic hydrocarbon group, more preferably a chain hydrocarbon group having 1 to 6 carbon atoms or an aromatic hydrocarbon group having 6 to 10 carbon atoms, particularly preferably a methyl group, an ethyl group, a n-propyl group, a n-butyl group, a phenyl group, a benzyl group or a tolyl group, and most preferably a methyl group or a phenyl group.

X may be any group selected from the group consisting of CO(OₘR²), SOₙ(OₘR²), PO(OₘR²)(OₗR³), CN or NO₂. When X is CO(OₘR²), m is preferably 1. When X is SOₙ(OₘR²), m is preferably 0 and n is preferably 2. When X is PO(OₘR²)(OₗR³), 1 is preferably 0 and m is preferably 1. Among these, X is preferably SOₙ(OₘR²), NO₂, CO(OₘR²), or CN, more preferably CO(OₘR²) or CN, and still more preferably CN.

Y may be any group selected from the group consisting of R⁴, OR⁴ and NR⁴R⁵. Among these, Y is preferably R⁴ or OR⁴ and more preferably R⁴.

### [Method for producing fluorine-containing pyridone compound]

Specific examples of the method for producing the fluorine-containing pyridone compound of the present invention include the following method (a) or (b).
(a) A method having a step of reacting a fluoroisobutylene derivative represented by the following formula (2) with a compound represented by the following formula (3) to obtain a fluorine-containing pyridone compound represented by the following formula (1). 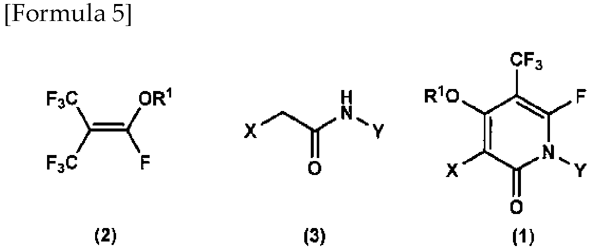 (In the formulae (1) to (3) above, X, Y and R¹ are as defined above.)
(b) A method having a step of reacting a fluoroisobutane derivative represented by the following formula (4) with a compound represented by the following formula (3) to obtain a fluorine-containing pyridone compound represented by the following formula (1).

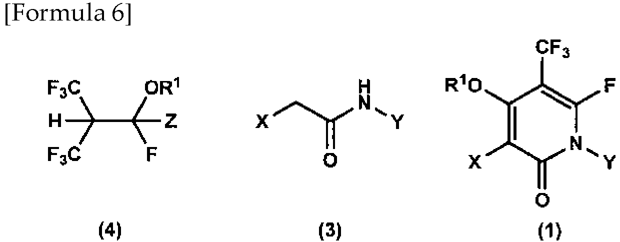

(In the formulae (1), (3) and (4) above, X, Y and R¹ are as defined above, Z represents a halogen atom, OCO(OₖR⁶) or OₖSOᵢ(OⱼR⁶),
R⁶ represents a hydrocarbon group having 1 to 10 carbon atoms,
j and k are each independently 0 or 1, and
i is 1 or 2.)

In general, methods for synthesizing a fluorine-containing compound are broadly divided into a method starting from a raw material that originally contains fluorine (building block method) and a method newly introducing fluorine into a compound. The former method is characterized in that the position where fluorine is introduced depends on a raw material. On the other hand, the latter method can only use a raw material having a substituent that can be co-present under the reaction conditions upon introduction of fluorine, and often necessitates preliminarily introducing a "marker" such as bromine or iodine at the position where fluorine is introduced, as a result of which the method is poor in efficiency. In the production method of the present invention, employing the method (a) or (b) above enables a novel fluorine-containing pyridone compound (the fluorine-containing pyridone compound of the present invention) to be obtained in which fluorine is introduced into a position where introduction has been considered difficult with conventional methods. The production method of the present invention is a building block method, which enables the fluorine-containing pyridone compound of the present invention to be efficiently obtained.

When Z is a halogen atom, the halogen atom is F, Cl, Br or **I,** preferably F or Cl, and more preferably F. When Z is OCO(OₖR⁶) or OₖSOᵢ(OⱼR⁶), R⁶ represents a hydrocarbon group having 1 to 10 carbon atoms. Examples of the hydrocarbon group having 1 to 10 carbon atoms include the hydrocarbon group having 1 to 10 carbon atoms among the hydrocarbon groups as described for R¹ to R⁵ above. When Z is OCO(OₖR⁶), k is preferably 0. When Z is OₖSOᵢ(OⱼR⁶), i is preferably 2, j is preferably 0, and k is preferably 1.

The compounds represented by the formulae (2), (3) and (4) above to be used may be commercially available or may be produced by a known method or the like.

In the methods (a) and (b) above, a reaction may be carried out in the presence of an organic solvent. Examples of the organic solvent that can be used include ethers such as tetrahydrofuran, diethyl ether, dioxane, monoglyme, diglyme, triglyme, and tetraglyme; aromatic hydrocarbons such as benzene, toluene, and xylene; nitriles such as acetonitrile; and aprotic polar solvents such as dimethylformamide, dimethylacetamide, methylpyrrolidone, dimethylethyleneurea, tetramethylurea, dimethylsulfoxide, and sulfolane.

In the methods (a) and (b) above, a reaction may be carried out in the presence of a basic substance. Examples of the basic substance that can be used include alkali metal/alkaline earth metal hydroxides such as sodium hydroxide, potassium hydroxide, calcium hydroxide, lithium hydroxide, magnesium hydroxide, and barium hydroxide; alkali metal/alkaline earth metal carbonates such as sodium carbonate, potassium carbonate, sodium hydrogen carbonate, and potassium hydrogen carbonate; metal hydrides such as sodium hydride, potassium hydride, and calcium hydride; tertiary amines such as trimethylamine, triethylamine, diisopropylethylamine, diazabicycloundecene, 7-methyl-1,5,7-triazabicyclo[4.4.0]dec-5-ene, 2-tert-butyl-1,1,3,3-tetramethylguanidine, and N,N-dimethylaniline; and phosphazene bases such as 2-tert-butylimino-2-diethylamino-1,3-dimethylperhydro-1,3,2-diazaphosphorine.

A reaction temperature in the methods (a) and (b) above is preferably -20°C or higher and lower than the boiling point temperature of an organic solvent, more preferably 0 to 50°C, and still more preferably 10 to 30°C. A reaction time in the methods (a) and (b) above is preferably 0.5 to 48 hours, more preferably 1 to 36 hours, and still more preferably 10 to 25 hours.

The above describes the embodiments of the present invention, but the present invention is not limited to the embodiments described above and includes all aspects included in the concept of the present invention and the scope of the claims, and can be modified in various ways within the scope of the present invention.

### Examples

The present invention will be described in more detail below with reference to Examples, but the present invention is in no way limited to these Examples.

### (Example 1)

### [Production of 3-cyano-6-fluoro-4-methoxy-1-phenyl-5-(trifluoromethyl)pyridin-2-one]

Under ice-water cooling, to 30 g of acetonitrile were added 1.0 g (6.2 mmol) of N-phenyl-2-cyanoacetamide and 1.5 g (7.1 mmol) of 1,3,3,3-tetrafluoro-1-methoxy-2-(trifluoromethyl)-1-propene. Subsequently, 5.1 g (19 mmol) of 2-tert-butylimino-2-diethylamino-1,3-dimethylperhydro-1,3,2-diazaphosphorine was added dropwise such that the internal temperature did not exceed 10°C, and the temperature was raised to room temperature. After about 16 hours, the contents were subjected to column purification to obtain 0.1 g of the compound represented by the following formula (chemical formula: C₁₄H₈F₄N₂O₂, molecular weight: 312.22 g/mol). The isolated yield thereof was 5%.

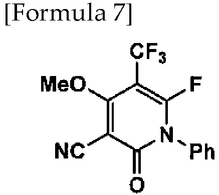

The analysis results were as follows.
Mass Spectrum (APCI, m/z): 312 ([M]⁺)

### (Example 2)

### [Production of 3-cyano-6-fluoro-4-methoxy-1-methyl-5-(trifluoromethyl)pyridin-2-one]

Under ice-water cooling, to 30 g of acetonitrile were added 1.0 g (10 mmol) of N-methyl-2-cyanoacetamide and 2.8 g (12 mmol) of 1,1,1,3,3-pentafluoro-3-methoxy-2-trifluoromethylpropane. Subsequently, 11 g (40 mmol) of 2-tert-butylimino-2-diethylamino-1,3-dimethylperhydro-1,3,2-diazaphosphorine was added dropwise such that the internal temperature did not exceed 10°C, and the temperature was raised to room temperature. After about 16 hours, the contents were subjected to column purification to obtain 80 mg of the compound represented by the following formula (chemical formula: C₉H₆F₄N₂O₂, molecular weight: 250.15 g/mol). The isolated yield thereof was 3%.

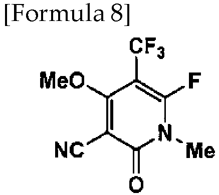

The analysis results were as follows.
Mass Spectrum (APCI, m/z): 250 ([M]⁺)
¹H-NMR (400 MHz, CDCl₃) δ ppm: 4.01 (s, 3H), 3.49 (s, 3H)

### (Example 3)

### [Production of 3-cyano-6-fluoro-4-methoxy-1-[(4-methoxyphenyl)methyl]-5-(trifluoromethyl)pyridin-2-one]

To 15 g of acetonitrile were added 0.5 g (2.5 mmol) of 2-cyano-N-[(4-methoxyphenyl)methyl]acetamide and 0.6 g (2.8 mmol) of 1,1,1,3,3-pentafluoro-3-methoxy-2-trifluoromethylpropane. Subsequently, 1.7 g (7.4 mmol) of tert-butylimino-tris(dimethylamino)phosphorane was added dropwise in an ice bath, and the temperature was raised to room temperature. After 17 hours, the contents were subjected to column purification to obtain a trace amount of a mixture of the compounds represented by the following formula (chemical formula: C₁₆H₁₂F₄N₂O₃, molecular weight: 356.28 g/mol).

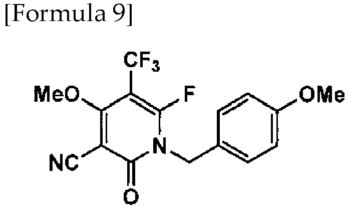

The analysis results were as follows.
Mass Spectrum (APCI, m/z): 355.7 ([M]⁺)

### (Example 4)

### [Production of 3-cyano-6-fluoro-4-methoxy-1-(phenylmethyl)-5-(trifluoromethyl)pyridin-2-one]

To 15 g of acetonitrile were added 0.5 g (2.9 mmol) of N-benzyl-2-cyanoacetamide and 0.7 g (3.3 mmol) of 1,1,1,3,3-pentafluoro-3-methoxy-2-trifluoromethylpropane. Subsequently, 2.0 g (8.7 mmol) of tert-butylimino-tris(dimethylamino)phosphorane was added dropwise in an ice bath, and the temperature was raised to room temperature. After 18 hours, the contents were subjected to column purification to obtain a trace amount of a mixture of the compounds represented by the following formula (chemical formula: C₁₅H₁₀F₄N₂O₂, molecular weight: 326.25 g/mol).

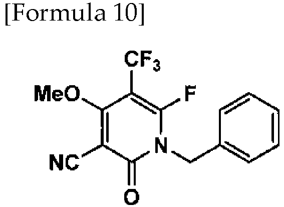

The analysis results were as follows.
Mass Spectrum (APCI, m/z): 325.9 ([M]⁺)

From those described above, the novel fluorine-containing pyridone compound of the present invention is found to enable production thereof without any problems.

### Industrial Applicability

The fluorine-containing pyridone compound of the present invention can be suitably used in the fields of pharmaceuticals and agrochemicals, and organic electronic materials science.

## Claims

1. A fluorine-containing pyridone compound represented by the following formula (1): 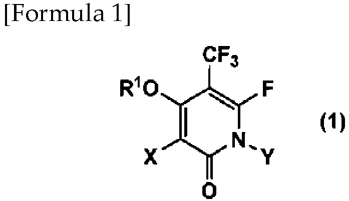 wherein
X represents CO(OₘR²), SOₙ(OₘR²), PO(OₘR²)(OₗR³), CN or NO₂,
Y represents R⁴, OR⁴ or NR⁴R⁵,
R¹ to R⁵ each independently represent a hydrocarbon group having 1 to 12 carbon atoms,
l and m are each independently 0 or 1, and
n is 1 or 2.

2. A fluorine-containing pyridone compound represented by the following formula (1): 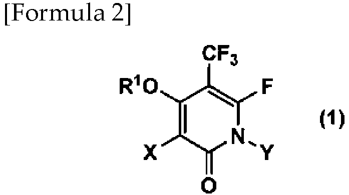 wherein
X represents CO(OₘR²), SOₙ(OₘR²), PO(OₘR²)(OₗR³), CN or NO₂,
Y represents a phenyl group, benzyl group, tolyl group, or naphthyl group to which one or more alkoxy groups having 1 to 5 carbon atoms are optionally bonded,
R¹ to R³ each independently represent a hydrocarbon group having 1 to 12 carbon atoms,
1 and m are each independently 0 or 1, and
n is 1 or 2.

3. A method for producing a fluorine-containing pyridone compound, comprising a step of reacting a fluoroisobutylene derivative represented by the following formula (2) with a compound represented by the following formula (3) to obtain a fluorine-containing pyridone compound represented by the following formula (1): 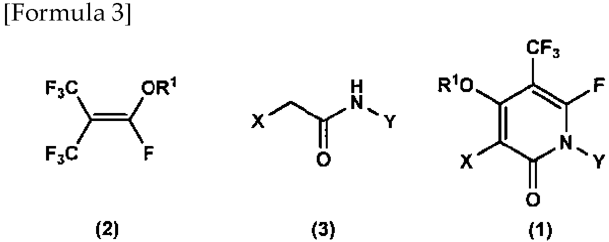 wherein
X represents CO(OₘR²), SOₙ(OₘR²), PO(OₘR²)(OₗR³), CN or NO₂,
Y represents R⁴, OR⁴ or NR⁴R⁵,
R¹ to R⁵ each independently represent a hydrocarbon group having 1 to 12 carbon atoms,
l and m are each independently 0 or 1, and
n is 1 or 2.

4. A method for producing a fluorine-containing pyridone compound, comprising a step of reacting a fluoroisobutane derivative represented by the following formula (4) with a compound represented by the following formula (3) to obtain a fluorine-containing pyridone compound represented by the following formula (1): 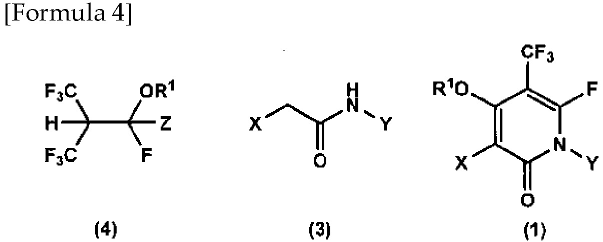 wherein
X represents CO(OₘR²), SOₙ(OₘR²), PO(OₘR²)(OₗR³), CN or NO₂,
Y represents R⁴, OR⁴ or NR⁴R⁵,
Z represents a halogen atom, OCO(OₖR⁶) or OₖSOᵢ(OⱼR⁶),
R¹ to R⁵ each independently represent a hydrocarbon group having 1 to 12 carbon atoms,
R⁶ represents a hydrocarbon group having 1 to 10 carbon atoms,
j, k, l, and m are each independently 0 or 1, and
i and n are each independently 1 or 2.
